# EUROPEAN PATENT APPLICATION

(11) **EP 4 275 678 A1**
(43) Date of publication of application: **15.11.2023**
(21) Application number: 21917810.0
(22) Date of filing: 19.01.2021
(51) Int. Cl.: A61K 9/20, A61K 31/5377, A61P 35/00

(54) **METHOD FOR PREPARING TABLET OF PHARMACEUTICAL COMPOSITION COMPRISING TRIAZOLOPYRAZINE DERIVATIVE COMPOUND AS ACTIVE INGREDIENT**

(30) Priority: 08.01.2021 KR 20210002315
(71) Applicant: Abion Inc., Seoul 08394 (KR)
(72) Inventor: CHOI, Jun Young, Gwangmyeong-si Gyeonggi-do 14315 (KR); PARK, Kyung Eui, Seoul 08061 (KR); KIM, Yeong Mun, Seoul 07017 (KR)
(74) Representative: Prüfer & Partner mbB Patentanwälte · Rechtsanwälte
(86) International application number: PCT/KR2021/000759
(87) International publication number: WO 2022/149647

(57) **Abstract**

The present invention relates to a tablet formulation composition comprising granules prepared from a triazolopyrazine derivative represented by formula 1 as a drug substance, an excipient, a binder and a disintegrant, and, more particularly, is an invention that solves problems in drug production caused by poor flowability and tabletability of the triazolopyrazine derivative represented by formula 1, and relates to a tablet formulation composition with improved stability formed by comprising mannitol as an excipient and low-substituted hydroxypropyl cellulose as a binder, and a method for preparing the same.

## Description

### Technical Field

The present invention relates to a method for preparing a pharmaceutical composition in a tablet formulation, comprising a triazolopyrazine derivative represented by the following formula 1 as an active ingredient.

### Background Art

The present invention relates to a method for preparing a pharmaceutical composition in a tablet formulation, comprising, as an active ingredient, a triazolopyrazine derivative (ABN401) represented by the following formula 1:

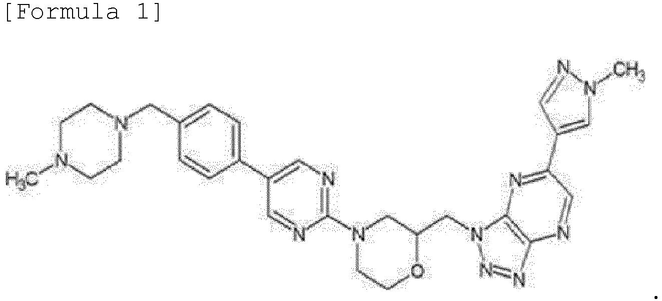

The inventors of the present invention discovered the above-described compound having inhibitory activities against tyrosine kinase and have diligently studied to develop a composition for efficiently preventing or treating various hyper proliferative disorders caused from the activities of abnormal tyrosine kinase.

A drug delivery system (DDS) refers to a technique of designing a drug formulation or a drug delivery method for selectively delivering a drug to a necessary part of a human body or a specific disease area to minimize side effects caused from the drug and maximize pharmacological effects. Such DDS may be classified into a sustained release (SR) system, an immediate release (IR) system, a controlled release (CR) system, a targeted DDS, and the like. The SR system is to design a formulation which slows down the release rate of a drug when the bioavailability is low, the absorption of the drug is too slow, or the rate of drug disappearance in vitro is high, so as to minimize problems. The IR system, which has a relatively short duration of drug release, as compared to the SR system, is to design a formulation which accelerates the release rate per unit time to increase the bioavailability and, thus, is used for cases where high drug efficiency is required in a short period of time.

The inventors of the present invention discovered triazolopyrazine, which is a compound having inhibitory activities against tyrosine kinase, and its derivatives, and found out that various hyper proliferative disorders caused from the activities of abnormal tyrosine kinase can efficiently be treated by using them. However, as a result of examining the physical properties of the triazolopyrazine and its derivatives, it was determined that the flowability and tabletability thereof were very poor due to the high content and many fine powders. When prepared as an immediate-release capsule composition, triazolopyrazine and its derivatives cause problems such as difficulty in tableting and non-uniform content due to the poor flowability and tableting properties. Accordingly, the researchers of the present invention confirmed that, when a tablet comprising the triazolopyrazine derivative compound represented by the above-described formula 1, microcrystalline cellulose (MCC) as the main component of a binder, low-substituted hydroxypropyl cellulose (LH21) as a disintegrant, and mannitol, phloxamer (Kolliphor P188), etc. as additives, had improved drug flowability, dissolution rate, blending uniformity, etc., was prepared, the drug flowability, dissolution rate and blending uniformity were improve, and have completed the present invention.

According to the present invention, the compound of the above-described formula 1 (ABN401) is known to bind to a hepatocyte growth factor (HGF) and inactivate phosphorylation, thereby significantly inhibiting the activities of c-Met kinase which triggers cell proliferation, migration and angiogenesis.

As used herein, the term "hyper proliferative disorder" refers to a pathological condition caused from excessive cell growth, division and migration that are not controlled by general restrictions in a normally growing animal body. The hyper proliferative disorders prevented or treated by the composition of the present invention comprise, for example, cancer, diabetic retinopathy, retinopathy of prematurity, corneal transplant rejection, neovascular glaucoma, erythema, proliferative retinopathy, psoriasis, rheumatoid arthritis, osteoarthritis, autoimmune diseases, Crohn's disease, restenosis, atherosclerosis, intestinal adhesion, ulcer, liver cirrhosis, glomerulonephritis, diabetic nephropathy, malignant nephrosclerosis, thrombotic microangiopathy, organ transplant rejection and glomerulopathy, but are not limited thereto, and comprise all hyper proliferative disorders caused from abnormal proliferation of cells and excessive generation of new blood vessels.

More preferably, cancer, which is one of the abnormal proliferative disorders that can be prevented and treated with the composition of the present invention, comprises lung cancer, stomach cancer, pancreatic cancer, colon cancer, ovarian cancer, renal cell cancer, prostate cancer or liver cancer.

### Prior Art Citation

U.S. Patent No. 8,507,487 B2

### Summary of Invention

### Technical Problem

The inventors of the present invention have developed appropriate excipients, binders and disintegrants in order to efficiently solve problems such as difficulty in tableting and non-uniform content due to poor flowability and tabletability of a triazolopyrazine compound, thereby aiming to provide a method for preparing a pharmaceutical composition in a tablet formulation, comprising a triazolopyrazine derivative represented by the above-described formula 1 as an active ingredient.

### Solution to Problem

In the process of exploring appropriate excipients, binders, diluents and disintegrants in order to efficiently solve problems such as difficulty in tableting and non-uniform content due to poor flowability and tabletability of the triazolopyrazine-based compound represented by formula 1, the inventors of the present invention confirmed that, when a tablet comprising mannitol as an excipient, microcrystalline cellulose (MCC) and low-substituted hydroxypropyl cellulose (L-HPC) as binders, and phloxamer (Killiphor P188) and sodium starch glycolate as disintegrants is prepared, it showed a dramatic effect in improving the drug flowability, dissolution rate and uniformity of drug release, and have completed the present invention.

### Advantageous Effects of Invention

The present invention solves problems such as difficulty in tableting and non-uniform content due to poor flowability and tabletability of the triazolopyrazine derivative (ABN401) represented by the above-described formula 1 by preparing a tablet comprising the additives provided in the present invention, thereby further improving the usefulness of the compound.

### Brief Description of Drawings

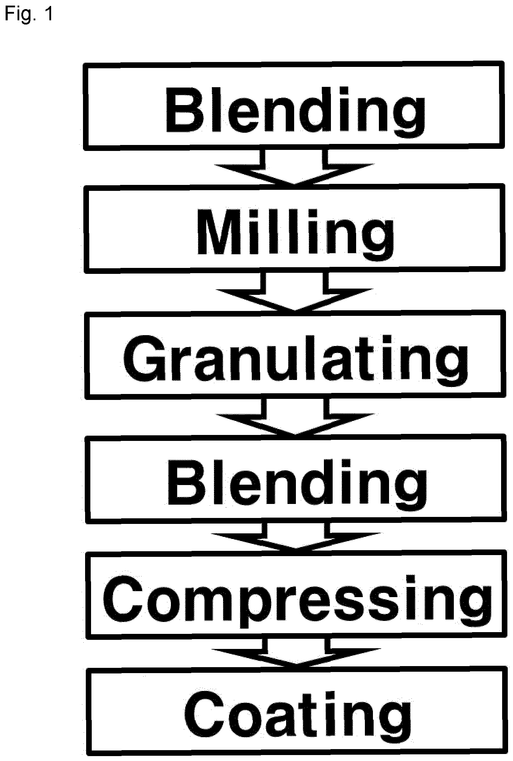

FIG. 1 is a schematic diagram of the process of preparing the tablet of the drug provided in the present invention.
FIG. 2 shows the results of dissolution of the drug of tablet provided in the present invention.
FIG. 3 shows the thickness and strength of the tablet provided in the present invention.

### Mode for Invention

Hereinafter, the present invention will be described in more detail through embodiments. However, the scope of the present invention is not limited to the embodiments.

The present invention relates to a method for preparing a pharmaceutical composition in a tablet formulation, comprising the above-described compound of formula 1 (ABN401) as an active ingredient, which can efficiently prevent or treat various hyper proliferative disorders by inhibiting the activities of c-Met kinase.

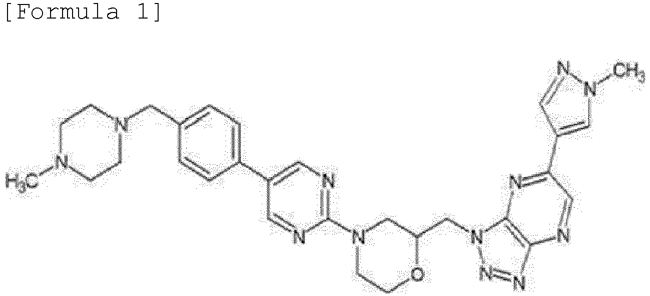

Embodiments described below may be modified in various forms, and the scope of the present invention is not limited to the embodiments described below. The embodiments of the present invention are provided to completely explain the present invention to those skilled in the art.

The present invention relates to a method for preparing a tablet of a pharmaceutical composition comprising a triazolopyrazine derivative (ABN401) represented by the above-described formula 1 as an active ingredient, and a tablet prepared by the method.

The method for preparing a tablet of a drug comprising the triazolopyrazine derivative as an active ingredient to be provided in the present invention comprises the following steps.

The method for preparing a tablet provided in the present invention specifically comprises:
a first step of blending and milling the triazolopyrazine derivative with a disintegrant, a binder and an excipient;
a second step of further adding a binder to the composition of the first step and passing through a granulation process to prepare granules;
a third step of adding and blending a binder solution to the composition prepared in the second step;
a fourth step of compressing the composition that has passed through the third step; and
a fifth step of coating the composition that has passed through the fourth step.

The binder used in the first and second steps of the present invention may comprise at least one component selected from the group consisting of microcrystalline cellulose, mannitol, low-substituted hydroxypropylcellulose (L-HPC, LH21), poloxamer (Kolliphor, P188), sodium stearyl fumarate, lactose monohydrate, sorbitol and sucrose.

In the present invention, the weight ratio of the triazolopyrazine derivative and the binder may be 20:0.1 to 50:5.

In the second step, a binder solution may be further added, and purified water may be used as the binder solution.

The disintegrant of the present invention may be at least any one selected from methylcellulose, crospovidone, alginic acid, croscarmellose, sodium starch glycolate and carboxymethylcellulose.

In the present invention, the weight ratio of the triazolopyrazine derivative and the disintegrant may be 20:0.1 to 50:5.

The tablet of the present invention may further comprise an excipient in addition to the binder and disintegrant.

The excipients used in the present invention are additives used in the development of drug formulations such as lubricants, surfactants and glidants, and may comprise at least any one selected from the group consisting of colloid silicon dioxide, magnesium trisilicate, corn starch, talc, carboxymethyl cellulose sodium, croscarmellose sodium, povidone K30, povidone K90, polyethylene glycol, polyethylene oxide, polyethylene propylene glycol copolymer, glyceryl monostearate, glyceryl palmitostearate, glyceryl behenate, ethyl maltol, wax, sodium acetate trihydrate, etc.

In the present invention, the weight ratio of the triazolopyrazine derivative and the excipient may be 25:55 to 50:70.

Opadry may be used as a coating agent used in the coating step, which is the fifth step of the preparation method provided in the present invention. The Opadry is a coating agent with proven stability that is widely used to coat drugs prepared in the solid form manufactured by Colorcon, and may improve hygroscopicity and light stability of tablets. The Opadry is prepared by blending polyvinyl alcohol, polyethylene glycol, titanium oxide, indigo carmine aluminum lake, etc., and, in addition to the above-described components, a colorant compound such as Brilliant Bluemay be added according to the color of the surface of the drug.

In the granulation process of the second step, a high shear granulator of Gerteis Minipactor was used. In the step of preparing granules by using the high shear granulator, the amount of the binder solution sprayed at an impeller speed of 400 rpm to 12000 rpm may be 25 ul/s to 75 ul/s, and, preferably, the amount of the binder solution sprayed at an impeller speed of 500 rpm to 12000 rpm may be 50 ul/s to 250 ul/s.

The step of drying the granules may be carried out in a fluid bed dryer. The fluid bed dryer is used for dry blending of powder, fine granules and granules of pharmaceuticals, chemicals and foods, and is a device that loads a material to be dried into a container, floats same to the top with heated air and dries same while flowing.

In the compression step, as the fourth step, a Beta Press SADE rotary tablet compressor was applied, and a pressure of 1 to 10 kN/cm may be applied.

In the coating step, as the fifth step, O'Hara Lab Coat was used, and a fan speed of 10 to 30 rpm may be applied.

Hereinafter, preferred embodiments are presented to aid understanding of the present invention. However, the following embodiments are only provided to more easily practice the present invention, and the scope of the present invention is not limited to the following embodiments.

### Example 1. Preparation of various tablets comprising ABN401 as an active ingredient

ABN401 used as an active ingredient in the present invention has a high content and many fine powders. Thus, when prepared as a tablet composition, ABN401 may have problems such as difficulty in tableting and non-uniform content. In order to overcome such problems, a screening experiment for selecting excipients that significantly affect the dissolution rate by using excipients (MCC, LM, LH21, CMC-Na) whose stability was secured through a mutual stability test between ABN401 and excipients was first carried out. According to the types and contents of excipients, binders, and excipients presented above, ABN401, excipients, binders and disintegrants were put into a high-shear granulator and blended at a rotational speed of 500 rpm for 5 minutes. While blending the mixture at 800 rpm for 4 minutes, 1 ml of purified water was added every 20 seconds until granules were formed to prepare granules. The prepared granules were dried in an oven at 60 °C for about 1 hour and 30 minutes.

The dried granules were sized by using a No. 25 sieve, and the type and content of the disintegrant of the post-blending described above were added according to the formulation amount and blended for about 5 minutes. Through the above-described process, the surface of the solid pharmaceutical composition prepared through four examples using ABN401 in the amount of 5mg, 25mg, 100mg and 200mg, respectively, was coated by using Opadry, and the exact amounts were measured and shown in the table below. Each number shown in the table below represents the content contained per tablet, and 30 tablets were prepared for each content corresponding to each example.

The contents of the components are listed in Table 1 below.

**[Table 1]**

| | Example 1 (5 mg) | Example 2 (25 mg) | Example 3 (100 mg) | Example 4 (200 mg) |
|---|---|---|---|---|
| ABN401 | 5.00 | 25.00 | 100.00 | 200.00 |
| Mannitol | 16.80 | 84.00 | 30.99 | 61.98 |
| MCC | 23.35 | 116.76 | 85.02 | 170.04 |
| L-HPC | 3.60 | 18.00 | 26.25 | 52.50 |
| Kolliphor | 0.60 | 3.00 | 3.00 | 6.00 |
| Sodium lauryl sulphate | 6.45 | 32.25 | - | - |
| Sodium Starch Glycolate | 3.3 | 16.50 | 48.75 | 97.50 |
| Colloidal silicon dioxide | 0.60 | 3.00 | 6.00 | 6.00 |
| Sodium Stearyl fumarate | 0.30 | 1.50 | 3.0 | 6.00 |
| Opadry | 1.80 | 9.0 | 9.0 | 18.00 |

### Experimental Example 1: Measurement of dissolution rate of tablets of Examples 1 to 4

In order to measure the dissolution rate of ABN401 dissolved from the tablets of Examples 1 to 4, the following were prepared.

The dissolution rate of the drug of the tablet of the present invention was measured by applying the HPLC method. To apply the HPLC method, 5N hydrochloric acid was first diluted in distilled water as an elution buffer solution to prepare 900ml of 0.1N hydrochloric acid solution and heated at 37 °C.

In order to measure the mobile phase of the drug by the HPLA method, TEA was diluted in a 20 mM ammonium carbonate solution at pH 9. 4 and an acetonitrile solvent to prepare 1 liter of 1% TEA solution, respectively, and the ammonium carbonate solution and TEA solution were blended at a volume ratio of 600:400.

In order to measure the HPLC mobile phase, 25 µl of each of the samples of Examples 1 to 4 was administered to the blended solution, and the paddle method specified in the dissolution test method of the Korean Pharmacopoeia was carried out. The absorbance was measured at 282 nm with a UV spectrometer (Jasco V-730 spectrohotometer) to carry out quantitative analysis on the dissolution amount of the drug at predetermined time points (i.e., 5minutes, 10minutes, 20minutes, 30minutes, 45minutes, infinity). The results of the dissolution test are shown in FIG. 2 and Table 2. All of the tablets prepared according to each example exhibited almost 100% dissolution within 20 minutes after the start of dissolution. Each number represents the percent dissolution rate.

**[Table 2]**

| Time (min) | Example 1 | | Example 2 | | Example 3 | | Example 4 | |
|---|---|---|---|---|---|---|---|---|
| | Mean | % RSD | Mean | % RSD | Mean | % RSD | Mean | % RSD |
| 5 | 89 | 5.7 | 100 | 3.1 | 37 | 19.2 | 23 | 8.8 |
| 10 | 110 | 4.9 | 104 | 2.0 | 82 | 20.0 | 45 | 6.5 |
| 20 | 109 | 5.3 | 105 | 2.1 | 103 | 1.5 | 88 | 7.0 |
| 30 | 108 | 5.1 | 106 | 2.1 | 105 | 0.9 | 101 | 1.8 |
| 45 | 105 | 5.2 | 106 | 2.0 | 105 | 0.8 | 102 | 2.5 |
| infinity | 105 | 5.4 | 106 | 2.0 | 105 | 0.7 | 104 | 1.6 |

From the above-described results, all of the tablets comprising the triazolopyrazine derivative represented by formula 1 prepared in the present invention as an active ingredient showed almost 100% dissolution within 20 minutes after the start of dissolution, which shows that the tablet provided in the present invention is suitable for use as an immediate release formulation.

### Experimental Example 2. Measurement of thickness and strength of ABN401 tablets of Examples 1 to 4

In order to confirm the physical stability of tablets 1 to 4 prepared in the above-described examples, the thickness and strength of each tablet were measured, and the results are shown in FIG. 3 and Table 3.

**[Table 3]**

| | Example 1 | Example 2 | Example 3 | Example 4 |
|---|---|---|---|---|
| Thickness (mm) | 2.45-2.65 | 3.76-12.90 | 3.96-4.42 | 5.33-5.62 |
| Strength(kP) | 3.30-9.90 | 3.63-7.00 | 4.80-14.70 | 13.50-24.50 |

The above-described examples and experimental examples showed that the tablet comprising the triazolopyrazine derivative represented by formula 1 provided in the present invention as an active ingredient was suitable as an immediate release drug formulation for oral administration, as confirmed by the dissolution rate, thickness and strength of the drug.

It is obvious to those skilled in the art that the technical idea of the present invention is not limited to the above-described examples and experimental examples, and covers all ranges that do not change the technical idea of the present invention.

### Industrial Applicability

The present invention is industrially applicable.

## Claims

1. A tablet formulation composition for the treatment of hyper proliferative disorders, comprising a triazolopyrazine derivative represented by the following formula 1: 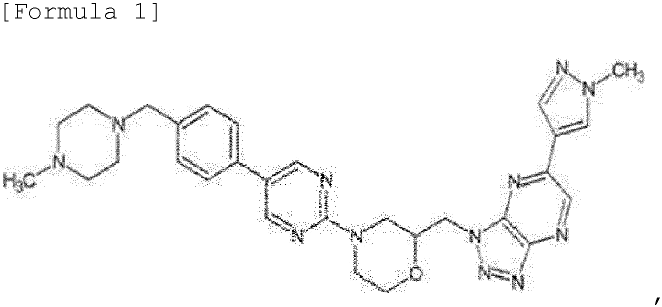 a binder, a disintegrant and an excipient;
wherein the weight ratio of the triazolopyrazine derivative and the binder is 20:0.1 to 50:5,
wherein the weight ratio of the triazolopyrazine derivative and the disintegrant is 20:0.1 to 50:5, and
wherein the weight ratio of the triazolopyrazine derivative and the excipient is 25:55 to 50:70.

2. The tablet formulation composition of claim 1, wherein the binder comprises at least any one selected from the group consisting of microcrystalline cellulose, mannitol, low-substituted hydroxypropylcellulose, poloxamer, sodium stearyl fumarate, lactose, starch, sorbitol and sucrose.

3. The tablet formulation composition of claim 1, wherein the disintegrant comprises at least any one selected from methylcellulose, crospovidone, alginic acid, croscarmellose, sodium starch glycolate and carboxymethylcellulose.

4. The tablet formulation composition of claim 1, wherein the excipient comprises at least any one selected from the group consisting of colloid silicon dioxide, magnesium trisilicate, corn starch, talc, carboxymethyl cellulose sodium, croscarmellose sodium, povidone, polyethylene glycol, polyethylenepropyleneglycolcopolymer,glycerylmonostearate, glyceryl palmitostearate, glyceryl behenate, ethyl maltol, wax and sodium acetate trihydrate.

5. The tablet formulation composition of claim 1, wherein the hyper proliferative disorder is any one selected from lung cancer, stomach cancer, pancreatic cancer, colon cancer, ovarian cancer, renal cell cancer, prostate cancer and liver cancer.

6. A method for preparing a tablet formulation composition for the treatment of a hyper proliferative disorder, comprising a triazolopyrazine derivative represented by the following formula 1: 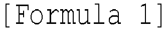 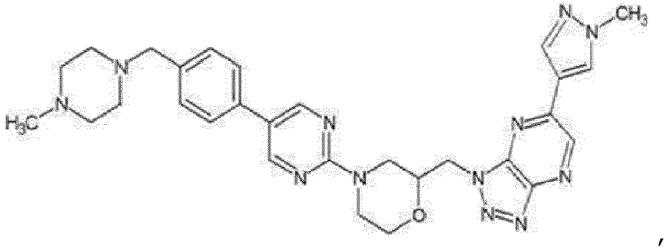
a binder, a disintegrant and an excipient,
wherein the method comprises the following steps:
a first step of blending and milling the triazolopyrazine derivative of formula 1 with a disintegrant, a binder and an excipient;
a second step of further adding a binder to the composition of the first step and passing through a granulation process to prepare granules;
a third step of adding and blending a binder solution to the composition prepared in the second step;
a fourth step of compressing the composition that has passed through the third step; and
a fifth step of coating the composition that has passed through the fourth step,
wherein the weight ratio of the triazolopyrazine derivative and the binder is 20:0.1 to 50:5,
wherein the weight ratio of the triazolopyrazine derivative and the disintegrant is 20:0.1 to 50:5, and
wherein the weight ratio of the triazolopyrazine derivative and the excipient is 25:55 to 50:70.

7. The method for preparing a tablet formulation composition of claim 6, wherein the binder comprises at least any one selected from the group consisting of microcrystalline cellulose, mannitol, low-substituted hydroxypropylcellulose, poloxamer, sodium stearyl fumarate, lactose, starch, sorbitol and sucrose.

8. The method for preparing a tablet formulation composition of claim 6, wherein the disintegrant comprises at least any one selected from methylcellulose, crospovidone, alginic acid, croscarmellose, sodium starch glycolate and carboxymethylcellulose.

9. The method for preparing a tablet formulation composition of claim 6, wherein the excipient comprises at least any one selected from the group consisting of colloid silicon dioxide, magnesium trisilicate, corn starch, talc, carboxymethyl cellulose sodium, croscarmellose sodium, povidone, polyethylene glycol, polyethylene propylene glycol copolymer, glyceryl monostearate, glyceryl palmitostearate, glyceryl behenate, ethyl maltol, wax and sodium acetate trihydrate.

10. The method for preparing a tablet formulation composition of claim 6, wherein the coating agent used in the coating step, which is the fifth step, is Opadry.
